# EUROPEAN PATENT SPECIFICATION

(11) **EP 4 551 122 B1**
(45) Date of publication and mention of the grant of the patent: **13.05.2026**
(21) Application number: 23735329.7
(22) Date of filing: 28.06.2023
(51) Int. Cl.: A61B 8/00, A61B 8/08

(54) **SYSTEM AND METHOD FOR PROCESSING ULTRASOUND IMAGING DATA**
SYSTEM UND VERFAHREN ZUM VERARBEITEN VON ULTRASCHALLBILDDATEN
SYSTÈME ET PROCÉDÉ DE TRAITEMENT DE DONNÉES D'IMAGERIE PAR ULTRASONS

(30) Priority: 04.07.2022 EP 22182823
(43) Date of publication of application: 14.05.2025
(73) Proprietor: Koninklijke Philips N.V., 5656 AG Eindhoven (NL)
(72) Inventor: WILD, Sebastian, 5656 AG Eindhoven (NL); EWALD, Arne, 5656 AG Eindhoven (NL); GOOSSEN, Andre, 5656 AG Eindhoven (NL); LOSSAU, Tanja, 5656 AG Eindhoven (NL); PETERS, Jochen, 5656AG Eindhoven (NL); GROTH, Alexandra, 5656 AG Eindhoven (NL); WAECHTER-STEHLE, Irina, 5656 AG Eindhoven (NL); WEBER, Frank Michael, 5656 AG Eindhoven (NL)
(74) Representative: Philips Intellectual Property & Standards
(86) International application number: PCT/EP2023/067636
(87) International publication number: WO 2024/008533

(56) References cited:
- WO-A1-2021/209348
- US-A1- 2011 079 082
- US-A1- 2022 008 041
- "Artificial Intelligence - Applications in Medicine and Biology", 27 April 2019 (2019-04-27), pages 1 - 20, XP055895748, ISBN: 978-1-78984-605-8, Retrieved from the Internet <URL:https://www.intechopen.com/chapters/63949> [retrieved on 20200702], DOI: 10.5772/intechopen.81628

## Description

### FIELD OF THE INVENTION

The invention relates to the field of ultrasound imaging.

### BACKGROUND OF THE INVENTION

When referring to ultrasound images, these can be acquired in either two or three dimensions. Two-dimensional (2D) ultrasound imaging remains a popular choice, as it allows a higher frame rate than three-dimensional imaging. In the case of contrast-enhanced ultrasound, two-dimensional ultrasound imaging results in less destruction of an injected contrast agent (also known as an ultrasound enhancing agent, UEA) compared with three-dimensional (3D) ultrasound imaging.

However, many automated image analysis algorithms are designed to operate on three-dimensional ultrasound images, and cannot be used to analyze two-dimensional ultrasound images.

There is therefore a need for an improved method for generating three-dimensional information from ultrasound imaging data.

WO 2021/209348 A1 discloses an ultrasound roadmap image generation system.

### SUMMARY OF THE INVENTION

The invention is defined by the claims.

### SUMMARY OF THE DISCLOSURE

According to examples in accordance with an aspect of the disclosure, there is provided a processing system for processing multiplane ultrasound imaging data. The processing system is configured to: obtain a first set of two-dimensional ultrasound images of an anatomical structure acquired, at a first time frame, by a multiplane ultrasound scanning device (e.g., an imaging probe), wherein at least two of the two-dimensional images have been acquired at a differently-oriented plane; obtain a three-dimensional ultrasound image of the anatomical structure acquired, at a third time frame, by a three-dimensional ultrasound scanning device, wherein the third time frame is different to the first time frame; and generate an estimated three-dimensional ultrasound image of the anatomical structure at the first time frame by processing at least the first set of two-dimensional ultrasound images and the three-dimensional ultrasound image of the anatomical structure using a machine-learning algorithm.

The first set of two-dimensional ultrasound images are multiplane images, i.e. images acquired/captured by an imaging probe at a same probe position and orientation and at/in a same time frame. The multiplane images (and therefore at least two of the images in the first set of two-dimensional ultrasound images) are differently-oriented to one another. In some examples, each image in the first set of two-dimensional ultrasound images has been acquired at a different-oriented plane. Preferably, one of the imaging axes of each multiplane image is at a same location and orientation (with respect to an imaged subject/individual) as an imaging axis of at least one other multiplane image. In other words, the multiplane images may share an imaging axis.

Preferably, each two-dimensional image in the first set (i.e., each multiplane image) comprises image data representing a same axis that extends from the probe that captured the mulitplane images into the subject/individual being imaged.

The inventors have recognized that multiplane imaging provides some three-dimensional information, and that the remaining information in a three-dimensional volume may be constructed by the use of a machine-learning algorithm.

The machine-learning algorithm is a machine-learning algorithm that has been trained to recognize three-dimensional features of an anatomical structure based on two-dimensional multiplane images. The generated estimated three-dimensional image is thus a "full" three-dimensional image, which may then be treated in the same way as a three-dimensional image obtained by three-dimensional imaging.

Since two-dimensional multiplane ultrasound imaging has a higher frame rate than three-dimensional ultrasound imaging, estimated three-dimensional ultrasound images may be produced at a higher frame rate than "real" three-dimensional ultrasound images. In addition to this, where a contrast (or enhancing) agent is used, two-dimensional multiplane ultrasound imaging will result in less destruction of the contrast agent, allowing a higher number of contrast-enhanced estimated three-dimensional ultrasound images to be produced.

The use of multiplane images, rather than two-dimensional images acquired sequentially by moving/rotating an imaging probe between acquisitions, means that the images are automatically spatially and temporally aligned. This avoids the introduction of errors when aligning images.

The temporal alignment is particularly valuable in applications that require a three-dimensional image from a particular point in time, such as cardiac imaging. Even when using cardiac gating, images from corresponding points of successive cardiac cycles will not align exactly, as different breathing states at the same point of the cardiac cycle, as well as irregularities in the cardiac cycle, will cause different heart shapes.

Preferably, the first set of two-dimensional ultrasound images comprises fewer than 10 images, e.g., 5 or fewer images. The proposed approach innovatively recognizes that it is possible to generate a 3D ultrasound image using only a restricted number of multiplane images through the use of a machine-learning algorithm.

The combination of a three-dimensional ultrasound image acquired at one time frame with two-dimensional multiplane ultrasound images acquired at a different time frame may improve an accuracy of the estimated three-dimensional ultrasound image, while not acquiring a full three-dimensional ultrasound image (using three-dimensional imaging) for every frame enables a higher frame rate. This may be particularly valuable for applications in which it is desirable to obtain three-dimensional imaging data over a period of time (e.g. over a cardiac cycle).

Further, in the case of contrast-enhanced ultrasound, acquiring two-dimensional multiplane ultrasound images at some time frames and three-dimensional images at other time frames allows three-dimensional imaging data to be generated for each time frame while reducing contrast agent destruction. This is particularly advantageous in contrast-enhanced cardiac ultrasound applications, such as left ventricular opacification, in which it is desirable to obtain contrast-enhanced three-dimensional images at each point throughout a complete cardiac cycle.

In some examples, the machine-learning algorithm has been trained using a training algorithm configured to receive a group of first training inputs and first known outputs, each first training input corresponding to a respective first known output. Each first training input comprises a set of simulated two-dimensional multiplane ultrasound images, wherein the set of simulated two-dimensional multiplane ultrasound images is generated from a three-dimensional ultrasound image. Each first known output comprises the three-dimensional ultrasound image used to generate the set of simulated two-dimensional multiplane ultrasound images for the corresponding first training input.

This allows a training set of training inputs and associated known outputs to be easily generated.

The set of simulated two-dimensional multiplane ultrasound images for each three-dimensional ultrasound image may be generated by resampling the three-dimensional image along given two-dimensional cut planes through the three-dimensional image.

In some examples, the machine-learning algorithm has been trained using a training algorithm configured to receive a group of second training inputs and second known outputs, each second training input corresponding to a respective second known output; each second training input comprises a set of two-dimensional multiplane ultrasound images of an anatomical structure for a subject: and each second known output comprises a three-dimensional ultrasound image of the anatomical structure represented by the set of two-dimensional multiplane ultrasound images of the corresponding second training input.

The sets of two-dimensional multiplane ultrasound images may be registered to the three-dimensional ultrasound images to form input-output pairs, each pair comprising a set of two-dimensional ultrasound images of an anatomical structure for a subject and a three-dimensional ultrasound image of the same anatomical structure for the same subject.

The terms "first" and "second" are used here purely to distinguish between the first and second training inputs and known outputs, and are not intended to imply that the second training inputs and known outputs require first training inputs and known outputs.

In some examples, the machine-learning algorithm is a generative adversarial network.

A generative adversarial network (GAN) may be used to ensure that the estimated three-dimensional ultrasound image is both consistent with the acquired two-dimensional multiplane ultrasound images and anatomically plausible. The GAN's discriminator network learns to distinguish between real three-dimensional ultrasound images and estimated three-dimensional ultrasound images generated from two-dimensional multiplane ultrasound images, while the generator network attempts to generate estimated three-dimensional ultrasound images that the discriminator network cannot distinguish from real three-dimensional ultrasound images.

In some examples, the processing system is further configured to provide the estimated three-dimensional ultrasound image to an image analysis algorithm developed for processing and/or analyzing three-dimensional imaging data.

As the estimated three-dimensional ultrasound image is a full three-dimensional image (rather than a set of multiplane images with gaps in-between), a "standard" image analysis algorithm designed for analyzing three-dimensional images can be used to analyze the estimated three-dimensional ultrasound image.

This allows a clinician to more efficiently make use of the three-dimensional information available in two-dimensional multiplane ultrasound images.

The image analysis algorithm may be a model-based segmentation algorithm.

Model-based segmentation algorithms produce more accurate results when applied to a three-dimensional image, as a three-dimensional object can be more accurately identified from a three-dimensional image than from a two-dimensional view.

In particular, it has been found that measurements derived from the segmentation of a two-dimensional image (such as measurements of cardiac parameters) are frequently inaccurate.

Providing an estimated three-dimensional image generated from two-dimensional multiplane images to a model-based segmentation algorithm designed for three-dimensional imaging data will therefore produce a more accurate segmentation than may be obtained by segmenting each of the multiplane images using an algorithm for two-dimensional images.

In some examples, the processing system is further configured to provide, at a display device, a visual representation of the estimated three-dimensional ultrasound image of the anatomical structure.

Displaying the estimated three-dimensional ultrasound image allows a clinician to more easily identify three-dimensional features, compared with simply displaying the two-dimensional multiplane ultrasound images.

The visual representation of the estimated three-dimensional ultrasound image may distinguish between regions of the image that comprise actually acquired data (i.e. the imaging data from the first set of two-dimensional ultrasound images) and estimated regions. For example, shading or a color overlay may be used to indicate the estimated regions.

In some examples, the processing system is configured to: obtain a second set of two-dimensional ultrasound images of the anatomical structure acquired, at a second time frame, by the multiplane ultrasound scanning device, wherein, in the second set, at least two of the two-dimensional ultrasound images have been acquired at a differently-oriented plane, and wherein the second time frame is different to the first time frame; and generate the estimated three-dimensional ultrasound image of the anatomical structure at the first time frame by processing at least the first set of two-dimensional ultrasound images and the second set of two-dimensional ultrasound images using the machine-learning algorithm.

Using additional imaging information, where available, may further improve an accuracy of the estimated three-dimensional image. Further sets of multiplane images may be obtained using a "spiral" acquisition mode, in which the orientations of the multiplane images rotate around a common axis over the acquisition period. Since these further images will typically be differently-oriented to the multiplane images of the first set as well as to one another, each set of multiplane images will provide some three-dimensional information not contained in the other set(s).

The output of the machine-learning algorithm may be a single estimated three-dimensional ultrasound image at the first time frame, or an estimated three-dimensional ultrasound image may be generated for each time frame at which a set of multiplane images has been acquired.

There is also provided an ultrasound system, comprising a multiplane ultrasound scanning device, and the processing system described above.

According to another aspect of the disclosure, there is provided a computer-implemented method for processing multiplane ultrasound imaging data. The computer-implemented method comprises: obtaining a first set of two-dimensional ultrasound images of an anatomical structure acquired, at a first time frame, by a multiplane ultrasound scanning device, wherein at least two of the two-dimensional ultrasound images have been acquired at a differently-oriented plane; obtaining a three-dimensional ultrasound image of the anatomical structure acquired, at a third time frame, by a three-dimensional ultrasound scanning device, wherein the third time frame is different to the first time frame; and generating an estimated three-dimensional ultrasound image of the anatomical structure at the first time frame by processing at least the first set of two-dimensional ultrasound images and the three-dimensional ultrasound image of the anatomical structure using a machine-learning algorithm.

In some examples, the computer-implemented method further comprises providing the estimated three-dimensional ultrasound image to an image analysis algorithm developed for processing and/or analyzing three-dimensional imaging data.

In some examples, the computer-implemented method further comprises providing, at a display device, a visual representation of the estimated three-dimensional ultrasound image of the anatomical structure.

In some examples, the computer-implemented method further comprises: obtaining a second set of two-dimensional ultrasound images of the anatomical structure acquired, at a second time frame, by the multiplane ultrasound scanning device, wherein, in the second set, at least two of the two-dimensional ultrasound images have been acquired at a differently-oriented plane, and wherein the second time frame is different to the first time frame; and wherein the step of generating the estimated three-dimensional ultrasound image of the anatomical structure at the first time frame comprises processing at least the first set of two-dimensional ultrasound images and the second set of two-dimensional ultrasound images using the machine-learning algorithm.

There is also proposed a computer program product comprising computer program code means which, when executed on a computer device having a processing system, cause the processing system to perform all of the steps of the method described above.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for acquiring and processing multiplane ultrasound imaging data, according to an embodiment of the invention; and
Fig. 2 illustrates a computer-implemented method 200 for processing multiplane ultrasound imaging data, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

There is proposed a system and method for processing multiplane ultrasound imaging data. A set of two-dimensional multiplane images of an anatomical structure, acquired near-simultaneously, is processed using a machine-learning algorithm to produce a synthetic three-dimensional image. The synthetic three-dimensional image is a full three-dimensional volume that is anatomically consistent with the input two-dimensional multiplane images.

Embodiments are at least partly based on the realization that multiplane images provide some three-dimensional information, and that training a machine-learning algorithm to estimate a full three-dimensional volume based on the three-dimensional information contained in the multiplane images enables the generation of three-dimensional images (which may then be input into an image analysis algorithm) at a higher frame rate than would be possible using three-dimensional imaging alone (i.e. using a three-dimensional scanning device), and, where applicable, with reduced contrast agent destruction.

Illustrative embodiments may, for example, be employed in ultrasound imaging systems (e.g. cardiac ultrasound imaging systems), and, in particular, in ultrasound image analysis applications, such as Philips QLAB.

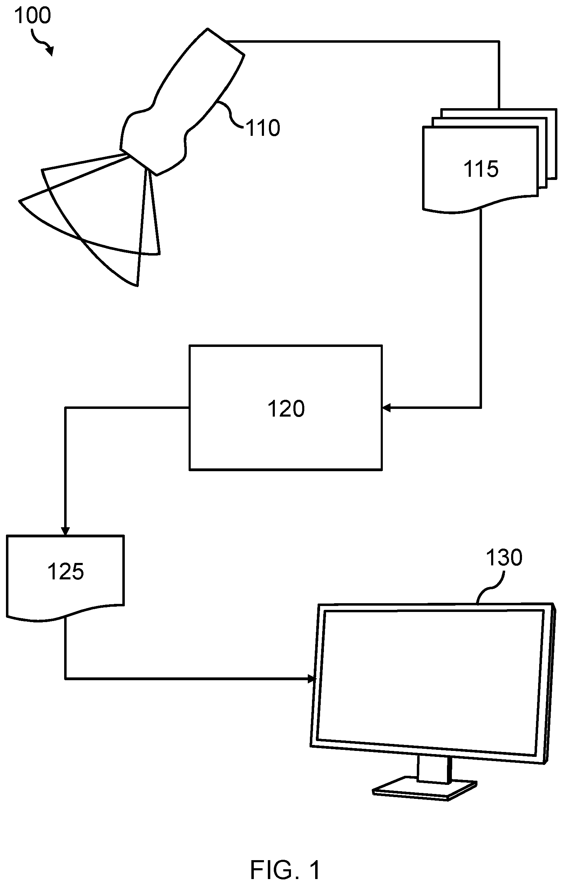

Fig. 1 illustrates a system 100 for acquiring and processing multiplane ultrasound imaging data, according to an embodiment of the invention. The system 100 comprises a multiplane ultrasound scanning device 110 and a processing system 120. The processing system is, itself, an embodiment of the invention.

The multiplane ultrasound scanning device may be any ultrasound scanning device capable of acquiring multiplane imaging data (i.e. an ultrasound scanning device comprising a two-dimensional array of transducers). For instance, the multiplane ultrasound scanning device may comprise a matrix array transducer.

The multiplane ultrasound scanning device 110 acquires, at a first time frame f₁, a first set of two-dimensional ultrasound images 115 of an anatomical structure, and the processing system 120 obtains the first set of two-dimensional ultrasound images from the multiplane scanning device.

The first set of two-dimensional ultrasound images 115 are multiplane images: that is, images acquired by an ultrasound scanning device having a same probe position and orientation. At least two of the images in the first set of two-dimensional ultrasound images are differently-oriented from each other. In some examples, each image in the first set may be differently-oriented. For example, the first set of two-dimensional images may consist of two orthogonal images.

Preferably, one of the imaging axes of each multiplane image is at a same location and orientation (with respect to an imaged subject/individual) as an imaging axis of at least one other multiplane image. In other words, the images forming the first set of two-dimensional ultrasound images may share a common imaging axis.

Preferably, each two-dimensional image in the first set (i.e., each multiplane image) comprises image data representing a same axis that extends from the probe that captured the mulitplane images into the subject/individual being imaged.

The first set of two-dimensional ultrasound images 115 are acquired by the multiplane scanning device at the same time frame f₁. Put another way, a time between acquisitions of consecutively-acquired ultrasound images in the first set is lower than a frame rate of the multiplane imaging. For instance, the time between consecutive acquisitions of images in the first set may be less than 20 ms. Preferably, the time between consecutive acquisitions of images in the first set may be less than 10 ms.

The time between consecutive acquisitions of the images in the first set 115 may be low enough that the anatomical structure imaged is unlikely to change significantly across the first set of two-dimensional multiplane images. For example, at a heart rate of 80 bpm, a time of 10 ms between consecutive acquisitions would correspond to just 1.3% of the cardiac cycle.

In Fig. 1, the multiplane ultrasound scanning device 110 is an X-plane ultrasound scanning device, so the first set of two-dimensional ultrasound images consists of two images. However, the invention is not limited to X-plane imaging, and the first set of two-dimensional ultrasound images may comprise more than two images.

Preferably, the first set of two-dimensional ultrasound images comprises fewer than 10 images, e.g., 5 or fewer images. The proposed approach innovatively recognizes that it is possible to generate a 3D ultrasound image using only a restricted number of multiplane images through the use of a machine-learning algorithm.

The processing system 120 processes the first set of two-dimensional images 115, using a machine-learning algorithm, to generate an estimated three-dimensional image 125 of the anatomical structure at the first time frame f₁.

A machine-learning algorithm is any self-training algorithm that processes input data in order to produce or predict output data. Here, the input data comprises at least the first set of two-dimensional images 115 and the output data comprises an estimated three-dimensional image 125 that corresponds to the first set of two-dimensional images. An estimated three-dimensional image is considered to correspond to a set of two-dimensional images if, for each two-dimensional image in the set, a two-dimensional cross-section of the estimated three-dimensional image having the same orientation and position as the two-dimensional image is identical, within a given accuracy, to the two-dimensional image.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

In some examples, the training input data entries may correspond to sets of simulated two-dimensional multiplane ultrasound images, wherein each set of simulated two-dimensional multiplane ultrasound images is generated from an example three-dimensional image. The training output data entries may correspond to the example three-dimensional images from which the training input data entries were generated. In other words, the training dataset may comprise input-output pairs, each pair comprising an example three-dimensional image of an anatomical structure and a set of simulated two-dimensional multiplane ultrasound images generated from the example three-dimensional image.

Methods of generating simulated two-dimensional ultrasound images from a three-dimensional ultrasound image will be apparent to the skilled person. For instance, each three-dimensional ultrasound image may be resampled along one or more two-dimensional cut planes through the image to produce the two-dimensional ultrasound image(s).

In other examples, the training input data entries may correspond to example sets of two-dimensional multiplane ultrasound images of an anatomical structure for a subject. The training output data entries may correspond to example three-dimensional ultrasound images, each of the same anatomical structure represented by a corresponding training input data entry.

Each example set of two-dimensional multiplane ultrasound images may be registered to the corresponding three-dimensional image. Methods of registering two-dimensional ultrasound images to three-dimensional space will be apparent to the skilled person. Each example set of two-dimensional multiplane ultrasound images may be acquired using the same ultrasound scanning device as the corresponding three-dimensional ultrasound image, at a same device position and orientation but operating in a different imaging mode, so that each input-output pair is automatically spatially aligned.

In addition to the estimated three-dimensional ultrasound image 125 corresponding to the input first set of two-dimensional ultrasound images 115, it is desirable that the machine-learning algorithm outputs an estimated three-dimensional ultrasound image that is anatomically plausible. Preferably, the estimated three-dimensional ultrasound image should resemble a real three-dimensional ultrasound image (i.e. an ultrasound image obtained using three-dimensional imaging) in terms of image features and appearance. This improves an accuracy of an output of an image analysis algorithm that has been developed for three-dimensional images, when the image analysis algorithm is provided with the estimated three-dimensional ultrasound image.

In some examples, this may be achieved by the use of a generative adversarial network (GAN). A generative adversarial network comprises a generator network and a discriminator network.

The generator network may be trained to generate an estimated three-dimensional ultrasound image from a training input (i.e. a set of two-dimensional multiplane ultrasound images obtained from multiplane imaging or simulated from a three-dimensional ultrasound image, as described above).

The discriminator network may be trained to distinguish real three-dimensional ultrasound images from estimated three-dimensional ultrasound images generated by the generator network. The discriminator may output a discriminator score, the value of which depends on how realistic an input three-dimensional ultrasound image is (with a higher score indicating a more realistic image).

The generator network and the discriminator network are trained together, with the generator network being modified according to the discriminator score given, by the discriminator network, to estimated three-dimensional ultrasound images generated by the generated network, and the discriminator network being modified according to whether the discriminator network correctly distinguished between real and estimated images. This leads to the generator network outputting more realistic estimated three-dimensional ultrasound images (i.e. estimated three-dimensional ultrasound images that will be given a high discriminator score by the discriminator network) and the discriminator network becoming increasingly more accurate.

This process may continue until a loss function, defined as the difference between a discriminator score for an estimated image and a discriminator score for a real image, is sufficiently close to zero (i.e. within a predetermined margin). At this stage, the generator network may be considered to have learnt to estimate three-dimensional ultrasound images that are indistinguishable from real images.

In some examples, the processing system 120 is configured to provide the estimated three-dimensional ultrasound image 125 to an image analysis algorithm developed for processing and/or analyzing three-dimensional imaging data.

The processing system 120 may automatically provide the estimated three-dimensional ultrasound image to a predetermined image analysis algorithm. Alternatively, the processing system may select one of a plurality of image analysis algorithms, and provide the estimated three-dimensional ultrasound image to the selected image analysis algorithm.

Examples of suitable image analysis algorithms include model-based segmentation algorithms and other algorithms that result in a measurement of an inherently three-dimensional quantity. Model-based segmentation algorithms for three-dimensional images typically produce more accurate segmentation results than model-based segmentation algorithms for two-dimensional images. For example, in the case of cardiac ultrasound imaging, a segmentation algorithm may be used to estimate left ventricular volume as a function of time. Since left ventricular volume is an inherently three-dimensional quantity, applying a segmentation algorithm to two-dimensional ultrasound images would not result in an accurate estimation of left ventricular volume.

In some examples, the system 100 further comprises a display device 130. The processing system 120 may be configured to provide a visual representation of the estimated three-dimensional ultrasound image 125 at the display device 130. Methods for providing visual representations of three-dimensional ultrasound images are well known, and will be apparent to the skilled person.

The visual representation may indicate which regions of the estimated three-dimensional image are based on actually acquired ultrasound imaging data (i.e. which regions were included in the first set of two-dimensional ultrasound images) and which regions have been estimated. For instance, shading or a color overlay may be used to distinguish between estimated regions and regions for which there is actual imaging data.

Where the estimated three-dimensional ultrasound image 125 has been provided to an image analysis algorithm, the processing system 120 may additionally or alternatively provide a visual representation of an output of the image analysis algorithm at the display device 130. For instance, where the estimated three-dimensional ultrasound image has been provided to a model-based segmentation algorithm, the display device may display a visual representation of the estimated three-dimensional ultrasound image with an overlay representative of the segmentation results.

In some examples, the multiplane scanning device 110 may acquire a second set of two-dimensional ultrasound images of the anatomical structure at a second time frame f₂, different to the first time frame f₁, and the processing system 120 may obtain the second set of two-dimensional ultrasound images from the multiplane scanning device.

The processing system 120 may then generate the estimated three-dimensional ultrasound image 125 of the anatomical structure at the first time frame f₁ by processing at least the first set of two-dimensional ultrasound images 115 and the second set of two-dimensional ultrasound images using the machine-learning algorithm.

At least two of the images in the second set of two-dimensional ultrasound images are differently-oriented from one another (e.g. each image in the second set may be differently-oriented), and one or more of the images in the second set may be differently oriented to each of the images in the first set. In this way, the second set of two-dimensional ultrasound images may provide additional anatomical information (albeit at a different time frame), which may be used to improve an accuracy of the estimated three-dimensional ultrasound image.

In order to generate an estimated three-dimensional ultrasound image using a first set of two-dimensional ultrasound images acquired at a first time frame f₁ and a second set of two-dimensional, training input data entries used to train the machine-learning algorithm may each comprise a first set of two-dimensional ultrasound images of an anatomical structure, as described above, and a second set of two-dimensional ultrasound images of the anatomical structure, acquired at a different time frame to the first set of two-dimensional ultrasound images. Where the first sets of two-dimensional ultrasound images in the training dataset are simulated images generated from a three-dimensional ultrasound image, the second set of two-dimensional images are each acquired at a different time to the three-dimensional image used to generate the corresponding first set of simulated two-dimensional images, or are themselves simulated images, generated from a three-dimensional image acquired at a different time to the three-dimensional image used to generate the corresponding first set of simulated two-dimensional images.

In some examples, the system 100 may further comprise a three-dimensional ultrasound scanning device. The three-dimensional ultrasound scanning device may be the same device as the multiplane ultrasound scanning device (operating in a three-dimensional imaging mode rather than a multiplane imaging mode), or a separate ultrasound scanning device (not shown in Fig. 1). The three-dimensional ultrasound scanning device may comprise a matrix array transducer.

The three-dimensional ultrasound scanning device may acquire a (real) three-dimensional ultrasound image of the anatomical structure at a third time frame f₃, different to the first time frame f₁, and the processing system 120 may obtain the real three-dimensional ultrasound image from the three-dimensional scanning device. A "real" three-dimensional image is an image of a full three-dimensional volume.

The processing system 120 may then generate the estimated three-dimensional ultrasound image 125 of the anatomical structure at the first time frame f₁ by processing at least the first set of two-dimensional ultrasound images 115 and the real three-dimensional ultrasound image using the machine-learning algorithm. A real three-dimensional ultrasound image acquired at the third time frame f₃ would provide anatomical information for regions not imaged by the multiplane scanning device at the first time frame f₁. This information may improve an accuracy of the estimated three-dimensional ultrasound image at the first time frame f₁, in particular because it would provide patient-specific information about the underlying 3D anatomy.

For example, in the case of cardiac ultrasound, a single real three-dimensional ultrasound image may be acquired by the three-dimensional scanning device at at least one point in the cardiac cycle, and a set of two-dimensional ultrasound images may be acquired by the multiplane scanning device at each of one or more other points in the cardiac cycle. Preferably the number of real three-dimensional ultrasound images acquired is low (for instance, only one real three-dimensional ultrasound image may be acquired during an imaging procedure or one real three-dimensional ultrasound image may be acquired for a given number of frames). For instance, in the case of cardiac ultrasound, a real three-dimensional image may be acquired twice per cardiac cycle (e.g. one real three-dimensional image may be acquired at end-diastole and another real three-dimensional image may be acquired at end-systole), with two-dimensional multiplane images being acquired at other points in the cardiac cycle.

An estimated three-dimensional ultrasound image at each of the one or more other points in the cardiac cycle may be generated by processing at least the real three-dimensional image(s) and the set of two-dimensional ultrasound images acquired at that point in the cardiac cycle using the machine-learning algorithm. This allows three-dimensional images to be produced (each either by three-dimensional imaging or output by the machine-learning algorithm) throughout the cardiac cycle accurately and at a higher frame rate than by acquiring real three-dimensional ultrasound images (i.e. using a three-dimensional ultrasound scanning device) for every frame.

In the case of contrast-enhanced cardiac ultrasound (e.g. left ventricular opacification), three-dimensional ultrasound imaging causes more destruction of an injected contrast agent than two-dimensional multiplane imaging. A combination of one or more real three-dimensional images and estimated three-dimensional images at time frames for which two-dimensional multiplane images were acquired allows accurate three-dimensional images while reducing the amount of contrast agent destruction.

In examples in which a real three-dimensional ultrasound image at a third time frame f₃ is used in addition to the first set of two-dimensional ultrasound images to generate the estimated three-dimensional ultrasound image, each training input data entry for the machine-learning algorithm may additionally comprise an example three-dimensional ultrasound image of the same anatomical structure as the corresponding set of two-dimensional ultrasound images, acquired by an scanning device at a different time to the set of two-dimensional ultrasound images (or, where the two-dimensional ultrasound images are simulated images generated from a three-dimensional image, at a different time to the three-dimensional image from which the simulated two-dimensional images were generated).

In some examples, the processing system 120 may generate the estimated three-dimensional ultrasound image 125 of the anatomical structure at the first time frame f₁ by processing, using the machine-learning algorithm, at least the first set of two-dimensional ultrasound images 115 acquired at the first time frame f₁, the second set of two-dimensional ultrasound images acquired at the second time frame f₂ and the real three-dimensional ultrasound image acquired at the third time frame f₃. It should be understood that the expressions "first time frame", "second time frame" and "third time frame" are used purely to differentiate between the time frames, and that the first set of two-dimensional ultrasound images, the second set of ultrasound images and the real three-dimensional ultrasound image may be acquired in any order.

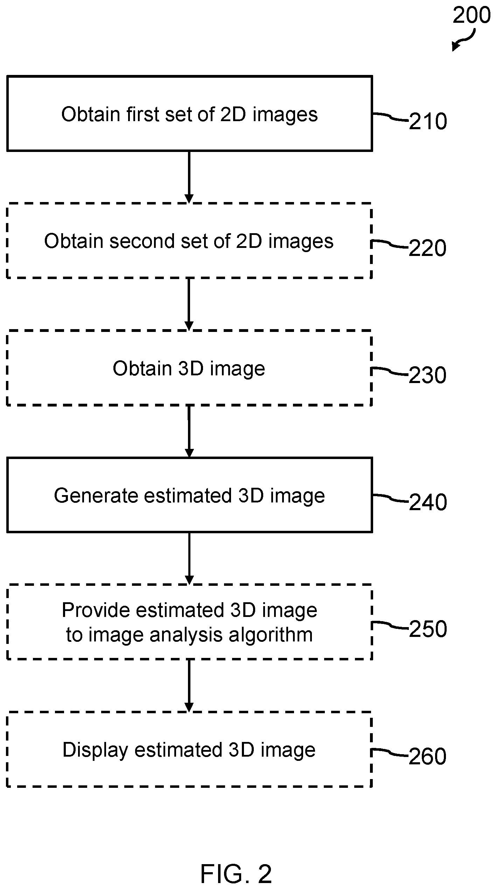

Fig. 2 illustrates a computer-implemented method 200 for processing multiplane ultrasound imaging data, according to an embodiment of the invention.

The method begins at step 210, at which a first set of two-dimensional ultrasound images of an anatomical structure are acquired, at a first time frame, by a multiplane ultrasound scanning device. At least two of the two-dimensional ultrasound images are acquired at a differently-oriented plane to one another.

At step 240, an estimated three-dimensional image of the anatomical structure at the first time frame is generated by processing at least the first set of two-dimensional images using a machine-learning algorithm.

In some examples, the method 200 further may comprise a step 250, at which the estimated three-dimensional ultrasound image is provided to an image analysis algorithm. The image analysis algorithm is an image analysis algorithm developed for processing and/or analyzing three-dimensional imaging data.

In some examples, the method 200 may further comprise a step 260, at which a visual representation of the estimated three-dimensional ultrasound image of the anatomical structure is provided at a display device.

In some examples, the method 200 may further comprise a step 220, at which a second set of two-dimensional ultrasound images of the anatomical structure are acquired, at a second time frame, by the multiplane scanning device. At least two of the two-dimensional ultrasound images in the second set are acquired at a differently-oriented plane. The second time frame is different to the first time frame. At step 240, the estimated three-dimensional image of the anatomical structure at the first time frame is then generated by processing at least the first set of two-dimensional images and the second set of two-dimensional ultrasound images using the machine-learning algorithm.

In some examples, the method 200 may further comprise a step 230, at which a three-dimensional ultrasound image of the anatomical structure is acquired, at a third time frame, by a three-dimensional scanning device. The third time frame is different to the first time frame. At step 240, the estimated three-dimensional image of the anatomical structure at the first time frame is then generated by processing at least the first set of two-dimensional images and the three-dimensional ultrasound image using the machine-learning algorithm.

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions. The processor may be located remotely (e.g. accessed using cloud infrastructure).

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A processing system (120) for processing multiplane ultrasound imaging data, the processing system being configured to:
obtain a first set of two-dimensional ultrasound images (115) of an anatomical structure acquired, at a first time frame (f₁), by a multiplane ultrasound scanning device (110), wherein at least two of the two-dimensional images have been acquired at a differently-oriented plane;
obtain a three-dimensional ultrasound image of the anatomical structure acquired, at a third time frame (f₃), by a three-dimensional ultrasound scanning device, wherein the third time frame is different to the first time frame (f₁); and
generate an estimated three-dimensional ultrasound image (125) of the anatomical structure at the first time frame by processing at least the first set of two-dimensional ultrasound images and the three-dimensional ultrasound image of the anatomical structure using a machine-learning algorithm,
wherein the machine-learning algorithm has been trained using a training algorithm configured to receive training input data entries and corresponding training output data entries,
wherein each training input data entry comprises a set of two-dimensional ultrasound images of an anatomical structure for a subject and each corresponding training output data entry comprises a three-dimensional ultrasound image of the same anatomical structure, and
wherein each training input data entry further comprises a three-dimensional ultrasound image of the same anatomical structure as the corresponding set of two-dimensional ultrasound images, said three-dimensional ultrasound image having been acquired at a different time to the set of two-dimensional ultrasound images of said training input data entry or to the three-dimensional image of the output data entry corresponding to said training input data entry.

2. The processing system of claim 1, wherein each two-dimensional image in the first set of two dimensional images has been acquired at a differently-oriented plane.

3. The processing system (120) of claim 1 or 2, wherein:
the training input data entries comprise a group of first training inputs and the training output data entries comprise first known outputs, each first training input corresponding to a respective first known output; and
the set of two-dimensional ultrasound images of each first training input comprises a set of simulated two-dimensional multiplane ultrasound images, wherein the set of simulated two-dimensional multiplane ultrasound images is generated from the three-dimensional ultrasound image of the respective first known output.

4. The processing system (120) of claim 1 or 2, wherein:
the training input data entries comprise second training inputs and the training output data entries comprise second known outputs, each second training input corresponding to a respective second known output;
the set of two-dimensional ultrasound images of each second training input comprises a set of two-dimensional multiplane ultrasound images of the anatomical structure; and
the three-dimensional ultrasound image of each second known output is represented by the set of two-dimensional multiplane ultrasound images of the corresponding second training input.

5. The processing system (120) of any of claims 1 to 4, wherein the machine-learning algorithm is a generative adversarial network.

6. The processing system (120) of any of claims 1 to 5, wherein the processing system is further configured to provide the estimated three-dimensional ultrasound image (125) to an image analysis algorithm developed for processing and/or analyzing three-dimensional imaging data.

7. The processing system (120) of claim 6, wherein the image analysis algorithm is a model-based segmentation algorithm.

8. The processing system (120) of any of claims 1 to 7, wherein the processing system is further configured to provide, at a display device (130), a visual representation of the estimated three-dimensional ultrasound image (125) of the anatomical structure.

9. The processing system (120) of any of claims 1 to 8, wherein the processing system is configured to:
obtain a second set of two-dimensional ultrasound images of the anatomical structure acquired, at a second time frame (f₂), by the multiplane ultrasound scanning device (110), wherein, in the second set, at least two of the two-dimensional ultrasound images have been acquired at a differently-oriented plane, and wherein the second time frame is different to the first time frame (f₁); and
generate the estimated three-dimensional ultrasound image (125) of the anatomical structure at the first time frame by processing at least the first set of two-dimensional ultrasound images (115) and the second set of two-dimensional ultrasound images using the machine-learning algorithm.

10. An ultrasound system, comprising:
a multiplane ultrasound scanning device; and
the processing system of any of claims 1 to 9.

11. A computer-implemented method (200) for processing multiplane ultrasound imaging data, the computer-implemented method comprising:
obtaining a first set of two-dimensional ultrasound images (115) of an anatomical structure acquired, at a first time frame (f₁), by a multiplane ultrasound scanning device (110), wherein at least two of the two-dimensional ultrasound images have been acquired at a differently-oriented plane;
obtaining a three-dimensional ultrasound image of the anatomical structure acquired, at a third time frame (f₃), by a three-dimensional ultrasound scanning device, wherein the third time frame is different to the first time frame (f₁); and
generating an estimated three-dimensional ultrasound image (125) of the anatomical structure at the first time frame by processing at least the first set of two-dimensional ultrasound images and the three-dimensional ultrasound image of the anatomical structure using a machine-learning algorithm,
wherein the machine-learning algorithm has been trained using a training algorithm configured to receive training input data entries and corresponding training output data entries,
wherein each training input data entry comprises a set of two-dimensional ultrasound images of an anatomical structure for a subject and each corresponding training output data entry comprises a three-dimensional ultrasound image of the same anatomical structure, and
wherein each training input data entry further comprises a three-dimensional ultrasound image of the same anatomical structure as the corresponding set of two-dimensional ultrasound images, said three-dimensional ultrasound image having been acquired at a different time to the set of two-dimensional ultrasound images of said training input data entry or to the three-dimensional image of the output data entry corresponding to said training input data entry.

12. The computer-implemented method (200) of claim 11, wherein the computer-implemented method further comprises:
obtaining a second set of two-dimensional ultrasound images of the anatomical structure acquired, at a second time frame (f₂), by the multiplane ultrasound scanning device (110), wherein, in the second set, at least two of the two-dimensional ultrasound images has been acquired at a differently-oriented plane, and wherein the second time frame is different to the first time frame (f₁);
and wherein the step of generating the estimated three-dimensional ultrasound image (125) of the anatomical structure at the first time frame comprises processing at least the first set of two-dimensional ultrasound images (115) and the second set of two-dimensional ultrasound images using the machine-learning algorithm.

13. A computer program product comprising computer program code means which, when executed on a computer device having a processing system, cause the processing system to perform all of the steps of the method (200) according to claim 11 or 12.

## Patentansprüche

1. Verarbeitungssystem (120) zum Verarbeiten von Mehrebenen-Ultraschallbildgebungsdaten, wobei das Verarbeitungssystem konfiguriert ist zum:
Erhalten eines ersten Satzes zweidimensionaler Ultraschallbilder (115) einer anatomischen Struktur, die in einem ersten Zeitrahmen (f₁) mit einer Mehrebenen-Ultraschallabtastvorrichtung (110) aufgenommen wurden, wobei mindestens zwei der zweidimensionalen Bilder in einer unterschiedlich orientierten Ebene aufgenommen wurden;
Erhalten eines dreidimensionalen Ultraschallbilds der anatomischen Struktur, die in einem dritten Zeitrahmen (f₃) mit einer dreidimensionalen Ultraschallabtastvorrichtung aufgenommen wurden, wobei sich der dritte Zeitrahmen vom ersten Zeitrahmen (f₁) unterscheidet; und
Generieren eines geschätzten dreidimensionalen Ultraschallbilds (125) der anatomischen Struktur im ersten Zeitrahmen durch Verarbeiten mindestens des ersten Satzes zweidimensionaler Ultraschallbilder und des dreidimensionalen Ultraschallbilds der anatomischen Struktur unter Verwendung eines maschinellen Lernalgorithmus,
wobei der maschinelle Lernalgorithmus unter Verwendung eines Trainingsalgorithmus trainiert wurde, der dazu konfiguriert ist, Trainingseingabedateneinträge und entsprechende Trainingsausgabedateneinträge zu empfangen,
wobei jeder Trainingseingabedateneintrag eine Reihe zweidimensionaler Ultraschallbilder einer anatomischen Struktur eines Subjekts umfasst und jeder entsprechende Trainingsausgabedateneintrag ein dreidimensionales Ultraschallbild derselben anatomischen Struktur umfasst, und
wobei jeder Trainingseingabedateneintrag ferner ein dreidimensionales Ultraschallbild derselben anatomischen Struktur wie der entsprechende Satz zweidimensionaler Ultraschallbilder umfasst, das dreidimensionale Ultraschallbild zu einem anderen Zeitpunkt als der Satz zweidimensionaler Ultraschallbilder des Trainingseingabedateneintrags oder als das dreidimensionale Bild des Ausgabedateneintrags, der dem Trainingseingabedateneintrag entspricht, aufgenommen wurde.

2. Verarbeitungssystem nach Anspruch 1, wobei jedes zweidimensionale Bild im ersten Satz zweidimensionaler Bilder in einer unterschiedlich orientierten Ebene aufgenommen wurde.

3. Verarbeitungssystem (120) nach Anspruch 1 oder 2, wobei:
die Trainingseingabedateneinträge eine Gruppe von ersten Trainingseingaben umfassen und die Trainingsausgabedateneinträge erste bekannte Ausgaben umfassen, wobei jede erste Trainingseingabe einer jeweiligen ersten bekannten Ausgabe entspricht; und
der Satz zweidimensionaler Ultraschallbilder jeder ersten Trainingseingabe einen Satz simulierter zweidimensionaler Mehrebenen-Ultraschallbilder umfasst, wobei der Satz simulierter zweidimensionaler Mehrebenen-Ultraschallbilder aus dem dreidimensionalen Ultraschallbild der jeweiligen ersten bekannten Ausgabe generiert wird.

4. Verarbeitungssystem (120) nach Anspruch 1 oder 2, wobei:
die Trainingseingabedateneinträge zweite Trainingseingaben umfassen und die Trainingsausgabedateneinträge zweite bekannte Ausgaben umfassen, wobei jede zweite Trainingseingabe einer jeweiligen zweiten bekannten Ausgabe entspricht;
der Satz zweidimensionaler Ultraschallbilder jeder zweiten Trainingseingabe einen Satz zweidimensionaler Mehrebenen-Ultraschallbilder der anatomischen Struktur umfasst; und
das dreidimensionale Ultraschallbild jeder zweiten bekannten Ausgabe durch den Satz zweidimensionaler Mehrebenen-Ultraschallbilder der entsprechenden zweiten Trainingseingabe dargestellt wird.

5. Verarbeitungssystem (120) nach einem der Ansprüche 1 bis 4, wobei der maschinelle Lernalgorithmus ein generatives adversariales Netzwerk ist.

6. Verarbeitungssystem (120) nach einem der Ansprüche 1 bis 5, wobei das Verarbeitungssystem ferner dazu konfiguriert ist, das geschätzte dreidimensionale Ultraschallbild (125) einem für die Verarbeitung und/oder Analyse dreidimensionaler Bildgebungsdaten entwickelten Bildanalysealgorithmus bereitzustellen.

7. Verarbeitungssystem (120) nach Anspruch 6, wobei der Bildanalysealgorithmus ein modellbasierter Segmentierungsalgorithmus ist.

8. Verarbeitungssystem (120) nach einem der Ansprüche 1 bis 7, wobei das Verarbeitungssystem ferner dazu konfiguriert ist, auf einer Anzeigevorrichtung (130) eine visuelle Darstellung des geschätzten dreidimensionalen Ultraschallbilds (125) der anatomischen Struktur bereitzustellen.

9. Verarbeitungssystem (120) nach einem der Ansprüche 1 bis 8, wobei das Verarbeitungssystem konfiguriert ist zum:
Erhalten eines zweiten Satzes zweidimensionaler Ultraschallbilder der anatomischen Struktur in einem zweiten Zeitrahmen (f₂) durch die Mehrebenen-Ultraschallabtastvorrichtung (110), wobei in dem zweiten Satz mindestens zwei der zweidimensionalen Ultraschallbilder in einer unterschiedlich orientierten Ebene aufgenommen wurden, und wobei sich der zweite Zeitrahmen von dem ersten Zeitrahmen (f₁) unterscheidet; und
Generieren des geschätzten dreidimensionalen Ultraschallbilds (125) der anatomischen Struktur im ersten Zeitrahmen durch Verarbeiten mindestens des ersten Satzes zweidimensionaler Ultraschallbilder (115) und des zweiten Satzes zweidimensionaler Ultraschallbilder unter Verwendung des maschinellen Lernalgorithmus.

10. Ultraschallsystem, umfassend:
eine Mehrebenen-Ultraschallabtastvorrichtung; und
das Verarbeitungssystem nach einem der Ansprüche 1 bis 9.

11. Computerimplementiertes Verfahren (200) zum Verarbeiten von Mehrebenen-Ultraschallbildgebungsdaten, wobei das computerimplementierte Verfahren Folgendes umfasst:
Erhalten eines ersten Satzes zweidimensionaler Ultraschallbilder (115) einer anatomischen Struktur, die in einem ersten Zeitrahmen (f₁) mit einer Mehrebenen-Ultraschallabtastvorrichtung (110) aufgenommen wurden, wobei mindestens zwei der zweidimensionalen Ultraschallbilder in einer unterschiedlich orientierten Ebene aufgenommen wurden;
Erhalten eines dreidimensionalen Ultraschallbilds der anatomischen Struktur, die in einem dritten Zeitrahmen (f₃) mit einer dreidimensionalen Ultraschallabtastvorrichtung aufgenommen wurden, wobei sich der dritte Zeitrahmen vom ersten Zeitrahmen (f₁) unterscheidet; und
Generieren eines geschätzten dreidimensionalen Ultraschallbilds (125) der anatomischen Struktur im ersten Zeitrahmen durch Verarbeiten mindestens des ersten Satzes zweidimensionaler Ultraschallbilder und des dreidimensionalen Ultraschallbilds der anatomischen Struktur unter Verwendung eines maschinellen Lernalgorithmus,
wobei der maschinelle Lernalgorithmus unter Verwendung eines Trainingsalgorithmus trainiert wurde, der dazu konfiguriert ist, Trainingseingabedateneinträge und entsprechende Trainingsausgabedateneinträge zu empfangen,
wobei jeder Trainingseingabedateneintrag eine Reihe zweidimensionaler Ultraschallbilder einer anatomischen Struktur eines Subjekts umfasst und jeder entsprechende Trainingsausgabedateneintrag ein dreidimensionales Ultraschallbild derselben anatomischen Struktur umfasst, und
wobei jeder Trainingseingabedateneintrag ferner ein dreidimensionales Ultraschallbild derselben anatomischen Struktur wie der entsprechende Satz zweidimensionaler Ultraschallbilder umfasst, das dreidimensionale Ultraschallbild zu einem anderen Zeitpunkt als der Satz zweidimensionaler Ultraschallbilder des Trainingseingabedateneintrags oder als das dreidimensionale Bild des Ausgabedateneintrags, der dem Trainingseingabedateneintrag entspricht, aufgenommen wurde.

12. Computerimplementiertes Verfahren (200) nach Anspruch 11, wobei das computerimplementierte Verfahren ferner Folgendes umfasst:
Erhalten eines zweiten Satzes zweidimensionaler Ultraschallbilder der anatomischen Struktur in einem zweiten Zeitrahmen (f₂) durch die Mehrebenen-Ultraschallabtastvorrichtung (110), wobei in dem zweiten Satz mindestens zwei der zweidimensionalen Ultraschallbilder in einer unterschiedlich orientierten Ebene aufgenommen wurden, und wobei sich der zweite Zeitrahmen von dem ersten Zeitrahmen (f₁) unterscheidet;
und wobei der Schritt zum Generieren des geschätzten dreidimensionalen Ultraschallbilds (125) der anatomischen Struktur im ersten Zeitrahmen Verarbeiten mindestens des ersten Satzes zweidimensionaler Ultraschallbilder (115) und des zweiten Satzes zweidimensionaler Ultraschallbilder unter Verwendung des maschinellen Lernalgorithmus umfasst.

13. Computerprogrammprodukt, das Computerprogrammcodemittel umfasst, die, wenn sie auf einer Computervorrichtung ausgeführt werden, die ein Verarbeitungssystem aufweist, das Verarbeitungssystem veranlassen, alle Schritte des Verfahrens (200) nach Anspruch 11 oder 12 durchzuführen.

## Revendications

1. Système de traitement (120) pour traiter des données d'imagerie ultrasonore multiplanaire, le système de traitement étant configuré pour :
obtenir un premier ensemble d'images ultrasonores bidimensionnelles (115) d'une structure anatomique acquises, à un premier instant (f₁), par un dispositif de balayage ultrasonore multiplanaire (110), dans lequel au moins deux des images bidimensionnelles ont été acquises dans un plan orienté différemment ;
obtenir une image ultrasonore tridimensionnelle de la structure anatomique acquise, à un troisième instant (f₃). par un dispositif de balayage ultrasonore tridimensionnel, dans lequel le troisième instant est différent du premier instant (f₁) ; et
générer une image ultrasonore tridimensionnelle estimée (125) de la structure anatomique au premier instant en traitant au moins le premier ensemble d'images ultrasonores bidimensionnelles et l'image ultrasonore tridimensionnelle de la structure anatomique à l'aide d'un algorithme d'apprentissage automatique,
dans lequel l'algorithme d'apprentissage automatique a été entraîné à l'aide d'un algorithme d'entraînement configuré pour recevoir des entrées de données d'entrée d'entraînement et des entrées de données de sortie d'entraînement correspondantes,
dans lequel chaque donnée d'entrée d'entraînement comprend un ensemble d'images ultrasonores bidimensionnelles d'une structure anatomique d'un sujet et chaque donnée de sortie d'entraînement correspondante comprend une image ultrasonore tridimensionnelle de la même structure anatomique, et
dans lequel chaque entrée de données d'entraînement comprend en outre une image ultrasonore tridimensionnelle de la même structure anatomique que l'ensemble correspondant d'images ultrasonores bidimensionnelles, ladite image ultrasonore tridimensionnelle ayant été acquise à un moment différent de celui de l'ensemble d'images ultrasonores bidimensionnelles de ladite entrée de données d'entraînement ou de l'image tridimensionnelle de l'entrée de données de sortie correspondant à ladite entrée de données d'entraînement.

2. Système de traitement selon la revendication 1, dans lequel chaque image bidimensionnelle du premier ensemble d'images bidimensionnelles a été acquise sur un plan orienté différemment.

3. Système de traitement (120) selon la revendication 1 ou 2, dans lequel :
les données d'entrée d'entraînement comprennent un groupe de premières entrées d'entraînement et les données de sortie d'entraînement comprennent des premières sorties connues, chaque première entrée d'entraînement correspondant à une première sortie connue respective ;
l'ensemble d'images ultrasonores bidimensionnelles de chaque première entrée d'entraînement comprend un ensemble d'images ultrasonores multiplanaires bidimensionnelles simulées, dans lequel l'ensemble d'images ultrasonores multiplanaires bidimensionnelles simulées est généré à partir de l'image ultrasonore tridimensionnelle de la première sortie connue respective.

4. Système de traitement (120) selon la revendication 1 ou 2, dans lequel :
les données d'entrée d'entraînement comprennent des deuxièmes entrées d'entraînement et les données de sortie d'entraînement comprennent des deuxièmes sorties connues, chaque deuxième entrée d'entraînement correspondant à une deuxième sortie connue respective ;
l'ensemble d'images ultrasonores bidimensionnelles de chaque deuxième entrée d'entraînement comprend un ensemble d'images ultrasonores multiplanaires bidimensionnelles de la structure anatomique ; et
l'image ultrasonore tridimensionnelle de chaque deuxième sortie connue est représentée par l'ensemble des images ultrasonores multiplanaires bidimensionnelles de la deuxième entrée d'entraînement correspondante.

5. Système de traitement (120) selon l'une quelconque des revendications 1 à 4, dans lequel l'algorithme d'apprentissage automatique est un réseau antagoniste génératif.

6. Système de traitement (120) selon l'une quelconque des revendications 1 à 5, dans lequel le système de traitement est en outre configuré pour fournir l'image ultrasonore tridimensionnelle estimée (125) à un algorithme d'analyse d'image développé pour le traitement et/ou l'analyse de données d'imagerie tridimensionnelle.

7. Système de traitement (120) selon la revendication 6, dans lequel l'algorithme d'analyse d'image est un algorithme de segmentation basé sur un modèle.

8. Système de traitement (120) selon l'une quelconque des revendications 1 à 7, dans lequel le système de traitement est en outre configuré pour fournir, sur un dispositif d'affichage (130), une représentation visuelle de l'image ultrasonore tridimensionnelle estimée (125) de la structure anatomique.

9. Système de traitement (120) selon l'une quelconque des revendications 1 à 8, dans lequel le système de traitement est configuré pour :
obtenir un deuxième ensemble d'images ultrasonores bidimensionnelles de la structure anatomique acquises, à un deuxième instant (f₂), par le dispositif de balayage ultrasonore multiplanaire (110), dans lequel, dans le deuxième ensemble, au moins deux des images ultrasonores bidimensionnelles ont été acquises dans un plan orienté différemment, et dans lequel le deuxième instant est différent du premier instant (f₁) ; et
générer l'image ultrasonore tridimensionnelle estimée (125) de la structure anatomique au premier instant en traitant au moins le premier ensemble d'images ultrasonores bidimensionnelles (115) et le deuxième ensemble d'images ultrasonores bidimensionnelles à l'aide de l'algorithme d'apprentissage automatique.

10. Système à ultrasons comprenant :
un dispositif de balayage ultrasonore multiplanaire ; et
le système de traitement selon l'une quelconque des revendications 1 à 9.

11. Procédé mis en œuvre par ordinateur (200) pour le traitement de données d'imagerie ultrasonore multiplanaire, le procédé mis en œuvre par ordinateur comprenant :
l'obtention d'un premier ensemble d'images ultrasonores bidimensionnelles (115) d'une structure anatomique acquises, à un premier instant (f₁), par un dispositif de balayage ultrasonore multiplanaire (110), dans lequel au moins deux des images ultrasonores bidimensionnelles ont été acquises dans un plan orienté différemment ;
l'obtention d'une image ultrasonore tridimensionnelle de la structure anatomique acquise, à un troisième instant (f₃), par un dispositif de balayage ultrasonore tridimensionnel, dans lequel le troisième instant est différent du premier instant (f₁) ;
la génération d'une image ultrasonore tridimensionnelle estimée (125) de la structure anatomique au premier instant en traitant au moins le premier ensemble d'images ultrasonores bidimensionnelles et l'image ultrasonore tridimensionnelle de la structure anatomique à l'aide d'un algorithme d'apprentissage automatique,
dans lequel l'algorithme d'apprentissage automatique a été entraîné à l'aide d'un algorithme d'entraînement configuré pour recevoir des entrées de données d'entrée d'entraînement et des entrées de données de sortie d'entraînement correspondantes,
dans lequel chaque donnée d'entrée d'entraînement comprend un ensemble d'images ultrasonores bidimensionnelles d'une structure anatomique d'un sujet et chaque donnée de sortie d'entraînement correspondante comprend une image ultrasonore tridimensionnelle de la même structure anatomique, et
dans lequel chaque entrée de données d'entraînement comprend en outre une image ultrasonore tridimensionnelle de la même structure anatomique que l'ensemble correspondant d'images ultrasonores bidimensionnelles, ladite image ultrasonore tridimensionnelle ayant été acquise à un moment différent de celui de l'ensemble d'images ultrasonores bidimensionnelles de ladite entrée de données d'entraînement ou de l'image tridimensionnelle de l'entrée de données de sortie correspondant à ladite entrée de données d'entraînement.

12. Procédé mis en œuvre par ordinateur (200) selon la revendication 11, dans lequel le procédé mis en œuvre par ordinateur comprend en outre :
l'obtention d'un deuxième ensemble d'images ultrasonores bidimensionnelles de la structure anatomique acquises, à un deuxième instant (f₂), par le dispositif de balayage ultrasonore multiplanaire (110), dans lequel, dans le deuxième ensemble, au moins deux des images ultrasonores bidimensionnelles ont été acquises dans un plan orienté différemment, et dans lequel le deuxième instant est différent du premier instant (f₁) ;
et dans lequel l'étape de génération de l'image ultrasonore tridimensionnelle estimée (125) de la structure anatomique au premier instant comprend le traitement au moins du premier ensemble d'images ultrasonores bidimensionnelles (115) et du deuxième ensemble d'images ultrasonores bidimensionnelles à l'aide de l'algorithme d'apprentissage automatique.

13. Produit de programme informatique comprenant des moyens de code de programme informatique qui, lorsqu'ils sont exécutés sur un dispositif informatique présentant un système de traitement, amènent le système de traitement à réaliser toutes les étapes du procédé (200) selon la revendication 11 ou 12.
